# EUROPEAN PATENT SPECIFICATION

(11) **EP 1 390 525 B1**
(45) Date of publication and mention of the grant of the patent: **13.08.2008**
(21) Application number: 02748716.4
(22) Date of filing: 23.05.2002
(51) Int. Cl.: C12Q 1/48, G01N 33/50, G01N 33/68

(54) **PYRUVATE-KINASE AS A NOVEL TARGET MOLECULE**
PYRUVAT-KINASE ALS EINEM NEUEN ZIELMOLEKÜL
PYRUVATE-KINASE UTILISEE COMME NOUVELLE MOLECULE CIBLE

(30) Priority: 23.05.2001 EP 01112601
(43) Date of publication of application: 25.02.2004
(73) Proprietor: Max-Planck-Gesellschaft zur Förderung der Wissenschaften e.V., 80539 München (DE)
(72) Inventor: ULLRICH, Axel, 80799 München (DE); STETAK, Attila, 85319 Starnberg (DE); KERI, Gyorgy, H-1021 Budapest (HU)
(74) Representative: Weiss, Wolfgang
(86) International application number: PCT/EP2002/005684
(87) International publication number: WO 2002/095063

(56) References cited:
- WO-A-98/35032
- MAZUREK S ET AL: "The role of phosphometabolites in cell proliferation, energy metabolism, and tumor therapy." JOURNAL OF BIOENERGETICS AND BIOMEMBRANES, vol. 29, no. 4, August 1997 (1997-08), pages 315-330, XP002940705 ISSN: 0145-479X
- AISAKI K ET AL: "Unique apoptotic changes in two Friend erythroleukemia cell lines with R-type pyruvate kinase gene mutation." BLOOD, vol. 90, no. 10 SUPPL. 1 PART 2, 15 November 1997 (1997-11-15), page 3B XP008001525 Thirty-ninth Annual Meeting of the American Society of Hematology;San Diego, California, USA; December 5-9, 1997 ISSN: 0006-4971
- NWANKWO J O: "Repression of cellular anaplerosis as the hypothesized mechanism of gamma-linolenic acid-induced toxicity to tumor cells." MEDICAL HYPOTHESES, vol. 56, no. 5, May 2001 (2001-05), pages 582-588, XP008001527 ISSN: 0306-9877
- AISAKI KEN-ICHI ET AL: "Apoptotic changes precede mitochondrial dysfunction in red cell-type pyruvate kinase mutant mouse erythroleukemia cell lines." JAPANESE JOURNAL OF CANCER RESEARCH, vol. 90, no. 2, February 1999 (1999-02), pages 171-179, XP008001530 ISSN: 0910-5050
- AIZAWA SHIN ET AL: "Effects of cyclic polylactate (CPL) on the growth of cloned leukemic cells in vitro." HEMATOLOGICAL ONCOLOGY, vol. 18, no. 2, June 2000 (2000-06), pages 51-60, XP008001526 ISSN: 0278-0232
- KANNO H ET AL: "Red-cell type pyruvate kinase as an anti-apoptotic molecule in erythroid progenitor cells." BLOOD, vol. 94, no. 10 SUPPL. 1 PART 1, 15 November 1999 (1999-11-15), page 194a XP002194132 Forty-first Annual Meeting of the American Society of Hematology;New Orleans, Louisiana, USA; December 3-7, 1999 ISSN: 0006-4971
- AISAKI KEN-ICHI ET AL: "Inhibition of glycolysis as well as mutations of pyruvate kinase gene causes apoptosis of erythroid progenitor cells both in vivo and vitro." BLOOD, vol. 96, no. 11 Part 1, 16 November 2000 (2000-11-16), page 596a XP002194133 42nd Annual Meeting of the American Society of Hematology;San Francisco, California, USA; December 01-05, 2000 ISSN: 0006-4971
- LUPULESCU AUREL: "Cancer cell metabolism: Its relevance to cancer treatment." CANCER INVESTIGATION, vol. 17, no. 6, 1999, pages 423-433, XP002214992 ISSN: 0735-7907
- DANG CHI V ET AL: "Oncogenes in tumor metabolism, tumorigenesis, and apoptosis." JOURNAL OF BIOENERGETICS AND BIOMEMBRANES, vol. 29, no. 4, August 1997 (1997-08), pages 345-354, XP008001528 ISSN: 0145-479X
- MAZUREK SYBILLE ET AL: "Effects of the human papilloma virus HPV-16 E7 oncoprotein on glycolysis and glutaminolysis: Role of pyruvate kinase type M2 and the glycolytic-enzyme complex." BIOCHEMICAL JOURNAL, vol. 356, no. 1, 2001, pages 247-256, XP002194134 ISSN: 0264-6021
- DATABASE BIOSIS [Online] BIOSCIENCES INFORMATION SERVICE, PHILADELPHIA, PA, US; November 1999 (1999-11) MAZUREK SYBILLE ET AL: "Energy metabolism in the involuting mammary gland." Database accession no. PREV200000187615 XP002194136 & IN VIVO (ATTIKI), vol. 13, no. 6, November 1999 (1999-11), pages 467-478, ISSN: 0258-851X
- WANG JUN ET AL: "Cytomegalovirus inhibits p53 nuclear localization signal function." JOURNAL OF MOLECULAR MEDICINE (BERLIN), vol. 78, no. 11, 2001, pages 642-647, XP002194135 ISSN: 0946-2716
- DATABASE BIOSIS [Online] BIOSCIENCES INFORMATION SERVICE, PHILADELPHIA, PA, US; 1994 MILAS LUKA ET AL: "Relation of apoptosis to cancer therapy." Database accession no. PREV199598143135 XP002194137 & IN VIVO (ATTIKI), vol. 8, no. 5, 1994, pages 665-673, ISSN: 0258-851X
- Pubmed abstract PMID: 8703074 Berg, DT et al, Science (1996) vol 273, no 5280, pp 1389-1391.
- Pubmed abstract PMID: 11598302 Mahal, LK et al, Science (2001) vol 294, no 5541, pp 380-381.

## Description

The present invention relates to the use of a pyruvate-kinase or a nucleic acid coding therefor as a target for the modulation of apoptotic processes, particularly as a target molecule for the diagnosis, prevention or treatment of apoptosis-associated disorders, such as tumours.

Pyruvate-kinase (ATP:pyruvate 2-O-phosphotransferase, PK), is a rate-controlling glycolytic enzyme and catalyses the formation of pyruvate and ATP from phosphoenolpyruvate and ADP. In mammals, PK exists as four isoenzymes named as M1-, M2-, L- and R-types the expression of which differ from one cell type to another (Tanaka et al. 1967, Ibsen et al. 1974, Nowak et al. 1981). The M2-type considered to be the prototype; is predominant in the foetus, in neuplasias and in undifferentiated or proliferating tissues, but also widely distributed in adult tissues (Mallati et al. 1992, Guminska et al. 1988). This form is progressively replaced by the M1-type in skeletal muscle, heart and brain during differentiation. Carcinogenesis apparently reverses this process. The M1-and M2-type are produced from the same gene by alternative splicing. The difference between these two isoforms is in one exon encoding 51 amino acid residues, in which 21 residues are different. The enzymological property of the M1-type isoenzyme is very different from the other three forms since it is the only one that is allosterically not regulated. The M1-type shows hyperbolic kinetics and is not activated by fructose-1,6-bisphosphate (FBP) in contrast, M2-type shows kinetic curve and is activated by FBP (Saheki et al. 1982).

The glycolytic enzyme glyceraldehyde-3-phosphate dehydrogenase exhibits functions unrelated to its glycolytic activity and plays a role in diverse cellular processes. For example GAPDH has been shown to translocate to the nucleus and induce apoptosis, to bind specific t-RNAs and possibly be involved in activation of transcription. There has, however, been no suggestion in the published literature that pyruvate-kinase is associated with apoptotic processes.

Thus, it was surprising that the anti-proliferative somatostatin analogue TT-232 (Kéri et al., 1993a, Kéri et al. 1993b and Kéri et al. 1996) binds to pyruvate-kinase, particularly pyruvate-kinase isoenzyme M2. The binding of TT-232 to pyruvate-kinase leads to a nuclear translocation and an induction of apoptosis. This result demonstrates that pyruvate-kinase is involved in apoptotic processes and represents a target molecule for the development of novel drugs, particularly of drugs against cancer and inflammatory diseases.

The present invention relates to a method of identifying novel modulators of apoptosis comprising screening for substances capable of modulating pyruvate-kinase activity, wherein the activity comprises a translocation to the nucleus.

A further aspect of the present invention relates to a nuclear pyruvate-kinase M2 isoform, which exhibits higher electrophoretic mobility compared to the cytosolic pyruvate-kinase isoform.

The term "pyruvate-kinase" as used in the present application refers to pyruvate-kinase isoenzyme M2. Particularly, the pyruvate-kinase is isoenzyme M2 (Swiss-Prot. number 14786). The DNA sequence encoding pyruvate kinase isoforms is disclosed in Genbank Accession number X56494. Pyruvate-kinases are obtainable from human or non-human animal sources, particularly from mammalian sources, such as man, mouse, rat, hamster, monkey, pig, etc. Especially preferred is the human M2-pyruvate-kinase comprising:
a) the amino acid sequence as shown in Swiss-Prot. number P14786 or
b) an amino acid sequence having an identity of at least 80%, particularly of at least 90% and more particularly of at least 95% thereto, wherein the amino acid sequence identity may be determined by a suitable computer-program, such as GCG or BLAST.

Furthermore, the term "pyruvate-kinase" encompasses recombinant derivatives or variants thereof, as well as fragments thereof having biological activity. These derivatives, variants and fragments thereof may be obtained as expression products from allelic variant genes or from recombinantly altered ones, e.g. modified or truncated genes and/or as products of protolytic cleavage. The term "biological activity" or "activity" in context with pyruvate-kinase preferably comprises the ability to modulate apoptosis, particularly the binding to the somatostatin analogue TT-223 and/or the translocation into the nucleus. Particularly important residues for pyruvate-kinase activity are the amino acid residues 334-420 containing a nuclear localisation signal.

A particularly preferred pyruvate-kinase variant is a nuclear pyruvate-kinase M2 isoform which differs from cytosolic pyruvate-kinase in that it exhibits a higher electrophoretic mobility. The nuclear isoform may be obtained from the cytosolic isoform by nuclear translocation and processing.

The pyruvate-kinase protein is encoded by a nucleic acid which may be a DNA or an RNA. Preferably, the nucleic acid comprises
(a) the nucleic acid sequence as shown in Genbank Accession number X56494 or complementary thereto
(b) a nucleic acid sequence corresponding to the sequence corresponding to (a) within the scope of degeneracy of the genetic code or
(c) a nucleic acid sequence hybridizing under stringent conditions with a sequence of (a) and/or (b).

The term "hybridization under stringent conditions" according to the present application is used as described in Sambrook et al., Molecular Cloning, A Laboratory Manual, Cold Spring Harbor, Laboratory Press (1989), 1.101-1.104. Consequently, hybridization under stringent conditions occurs when a positive hybridization signal is still detected after washing for 1 h with 1 x SSC and 0.1% SDS at 55°C, preferably at 62°C and most preferably 68°C, in particular for 1 h in 0.2 x SSC and 0.1 % SDS at 55°C, preferably at 62°C and most preferably at 68°C. A nucleotide sequence hybridizing under such washing conditions with a sequence as shown in the sequence listing or a complementary nucleotide sequence or a sequence within the scope of degeneracy of the genetic code is encompassed by the present invention.

The nucleic acid molecules of the invention may be recombinant nucleic acid molecules generated by recombinant methods, e.g. by known amplification procedures such as PCR. On the other hand, the nucleic acid molecules can also be chemically synthesized nucleic acids. Preferably, the nucleic acid molecules are present in a vector, which may be any prokaryotic or eukaryotic vector, on which the nucleic acid sequence is present preferably under control of a suitable expression signal, e.g. promoter, operator, enhancer etc. Examples of prokaryotic vectors are chromosomal vectors such as bacteriophages and extrachromosomal vectors such as plasmids, wherein circular plasmid vectors are preferred. Examples of eukaryotic vectors are yeast vectors or vectors suitable for higher cells, e.g. insect cells or mammalian cells, plasmids or viruses.

The pyruvate-kinase is capable of binding somatostatin analogue TT-232 and mediating its effect, particularly its effect to stimulate apoptotic processes. Thus, a stimulation of apoptosis may be obtained by increasing the amount and/or activity of pyruvate-kinase, particularly the nuclear translocation activity. In contrast thereto, an inhibition of apoptosis may be obtained by reducing the activity of pyruvate-kinase, particularly the nuclear translocation activity.

Due to its activity, pyruvate-kinase is a suitable target for agents for the diagnosis, prevention or treatment of an apoptosis-associated disorder, particularly a hyperproliferative disease which may be selected from tumours, e.g. colon cancer, breast cancer or melanomas and inflammatory diseases, e.g. neuronal inflammatory diseases, such as neuroarthritis, or a degenerative disease.

The present invention refers to a method of identifying novel modulators of apoptosis comprising screening for substances capable of modulating pyruvate-kinase M2 activity. Particularly, the invention relates to a method of identifying substances capable of binding to pyruvate-kinase and stimulating translocation to the nucleus.

The screening method may be a high throughput screening assay, wherein a plurality of substances is tested in parallel. The screening assay may be a cellular assay or a molecular assay, wherein an interaction of a substance to be tested with pyruvate-kinase activity is determined. Pyruvate-kinase may be provided in a cellular system, preferably in a cellular system overexpressing pyruvate-kinase, e.g. a cell culture or a non-human animal model. On the other hand, non-cellular systems may be used, e.g. pyruvate-kinase containing cell fractions or substantially isolated and purified pyruvate-kinase. Any active substance identified by this method, e.g. any substance capable of modulating, particularly stimulating nuclear translocation of pyruvate-kinase may be used as a pharmaceutical agent or as a lead structure which is further modified to improve pharmaceutical properties. Thus, the invention further comprises formulating a pyruvate-kinase modulator, as identified by the method as described above or a substance obtained therefrom, e.g. by chemical derivatization or by molecular modelling, as a pharmaceutical composition together with pharmaceutically acceptable carriers, diluents or adjuvants.

The present invention is explained in more detail in the following Figures and Examples:

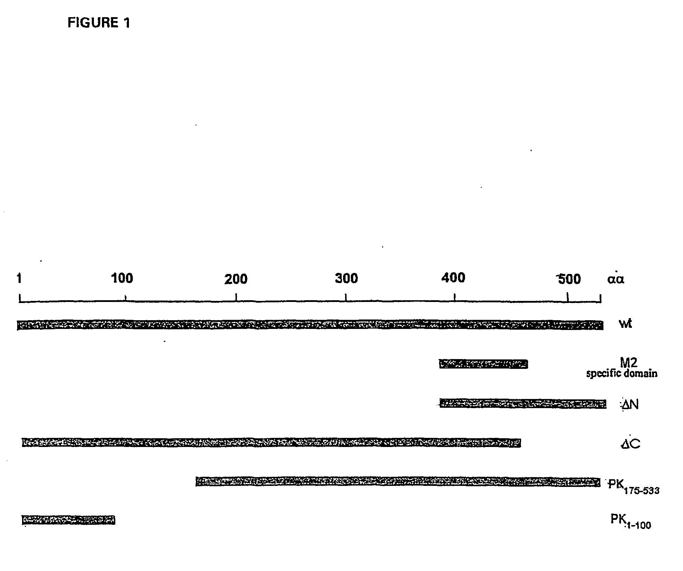

**Figure 1****.** Schematic representation of the generated pyruvate-kinase mutants.

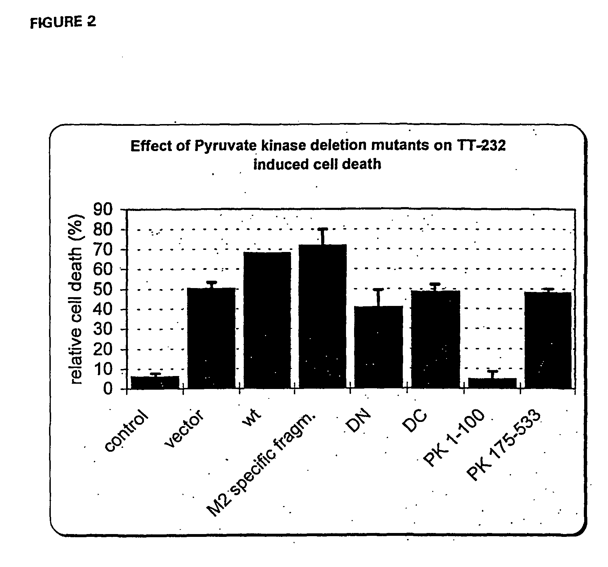

**Figure 2****.** Effect of different pyruvate-kinase deletion mutants on TT-232 induced cell death.

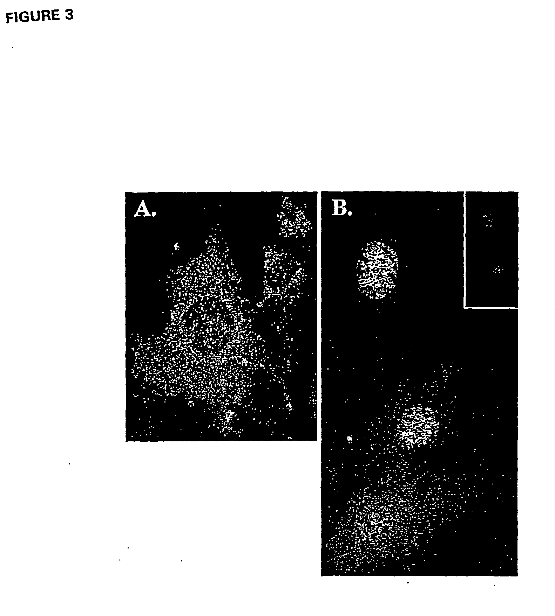

**Figure 3****.** Localisation of pyruvate-kinase. NIH3T3 cells were untreated (A) or treated with 50 µM TT-232 for 4 hours (B). Cells were fixed with methanol, blocked with PBS+3% BSA and subjected to immuno-staining with polyclonal PK-M2 specific primary and FITC labelled secondary antibody (green). Nuclei were visualized with propidium-iodide staining (red, insert picture).

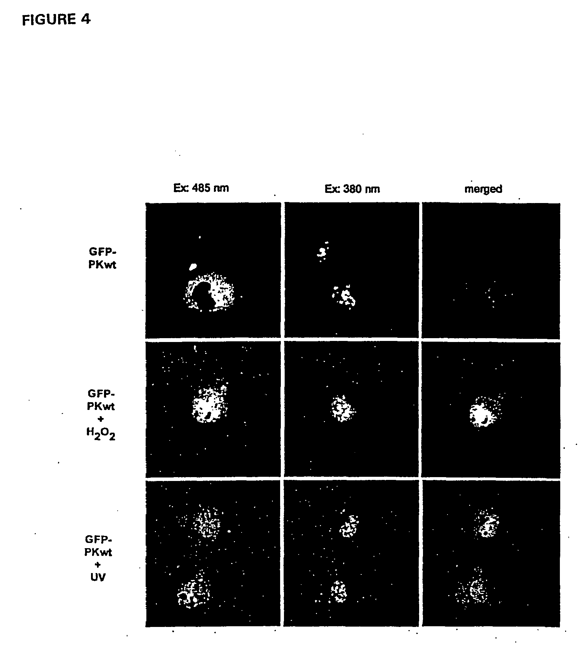

**Figure 4****.** Nuclear translocation of the pyruvate-kinase upon different apoptotic stimuli. Cos-7 cells were transiently transfected with pEGFP-PK-M2 eukaryotic expression plasmid (producing wild type pyruvate-kinase N-terminally fused to GFP)

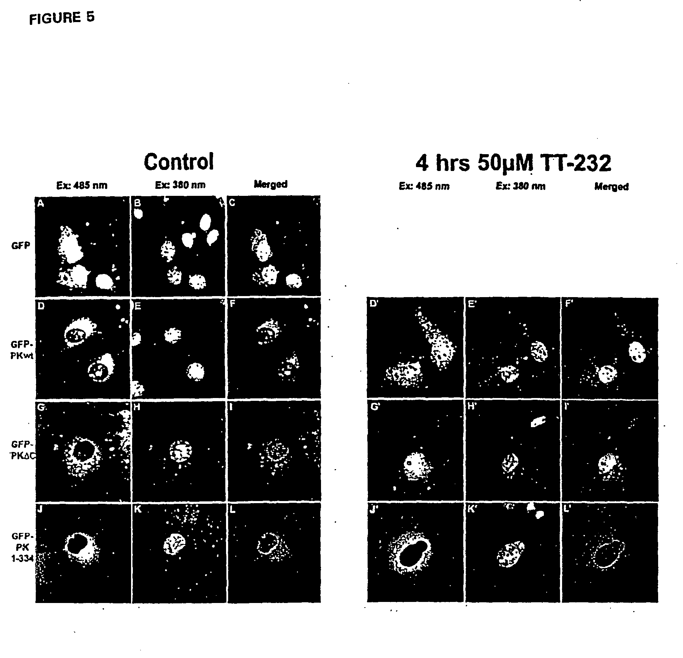

**Figure 5****.** Nuclear translocation of the pyruvate-kinase deletion mutants upon apoptotic stimuli. Cos-7 cells were transiently transfected with pEGFP-PK-M2 eukaryotic expression plasmid (producing different deletion mutants of the pyruvate-kinase N-terminally fused to GFP) and 48 hours after transfection cells were untreated (A-L) or treated with 50 *µ*M TT-232 for 4 hours (D'-L'). (Ex: 485 nm = GFP, Ex: 390 = propidium-iodide)

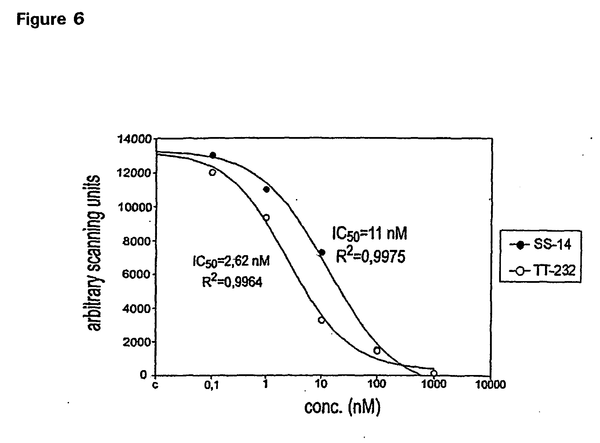

**Fig. 6****.** In vitro binding of somatostatin and TT-232 to rabbit muscle pyruvate-kinase.

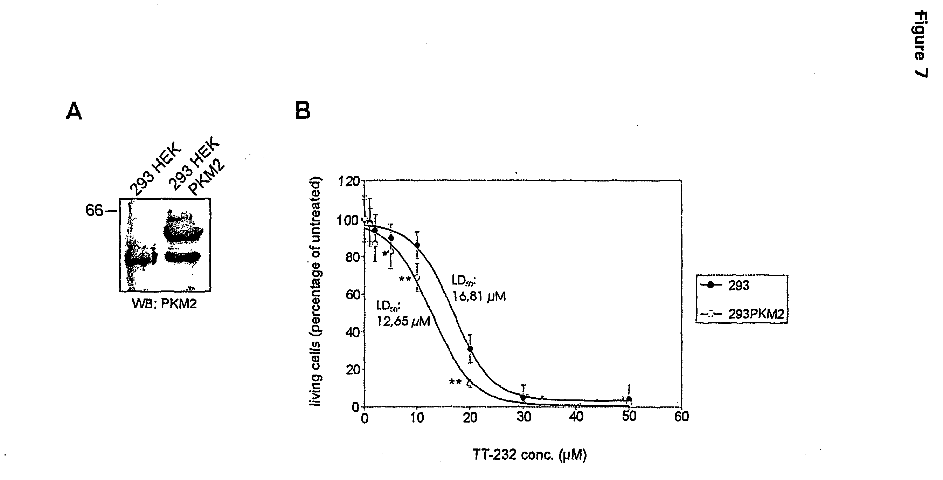

**Fig. 7****.** Effect of overexpression of pyruvate-kinase on sensitivity towards TT-232. (A) Western-blot of total cell lysates from 293HEK with or without overexpressing HA-tagged wild type PK-M2. (B) stably transfected cells were treated with increasing concentration of TT-232 for 24 hours and the amount of living cells was measured by MTT assay. (n = 32, *: p < 0.005, **: p<0.001 determined by student's T-test)

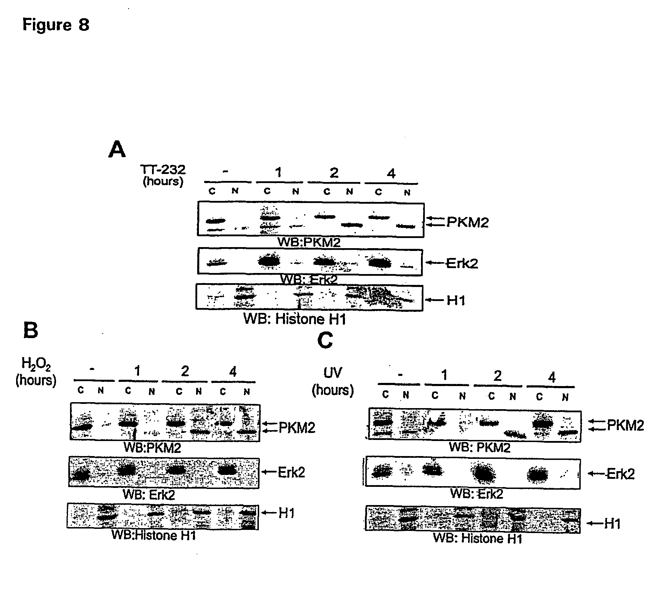

**Fig. 8****.** Subcellular localisation of endogenous PK-M2 upon peroxide or UV stimulation. Cos-7 cells were untreated, treated with 50 µM TT-232 (A), 100 µM hydrogen-peroxide (B), or 120 mJ/cm² UV (C) and incubated for different times as indicated. Cells were separated into cytosolic (c) or nuclear (n) fractions loaded in triplicate and blotted with PK-M2 (uppest panels), Erk-2 (middle panels) or Histone H1 (lowest panels).

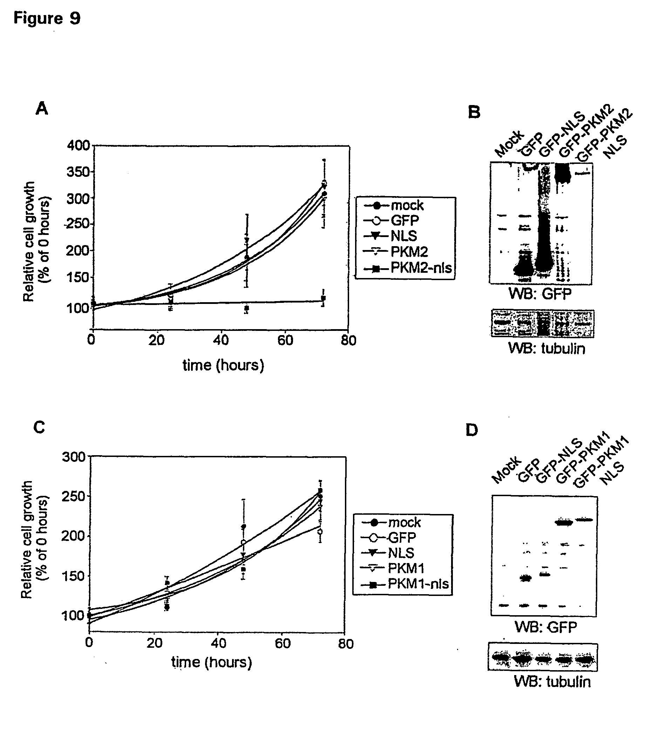

**Fig. 9****.** Effect of nuclear targeted PK-M2 on cell growth. (A and C) Cos-7 cells were transfected for 5 hours with empty vectors, pEGFP-PK-M2 (PKM2) or with pEYFP-PKM2-NLS (PKM2-NLS) (A) or pEGFP-PK-M1 (PKM2) or with pEYFP-PKM1-NLS (D). Cells were trypsinized, plated into 96 well plates, incubated for 24, 48, 72 hours in FCS containing medium, and cell density was measured with MTT assay. Cells were let to adhere for 10 hours, were measured and this was considered as zero time-point (n = 12 of three independent experiments). (B and D) A portion of the transfected cells was incubated for 48 hours, separated by 6-15% SDS-gradient gel electrophoresis and analysed in Western-blot with GFP antibody (upper panel). Reprobing the filter with tubulin antibody (lower panel) showed equal loading.

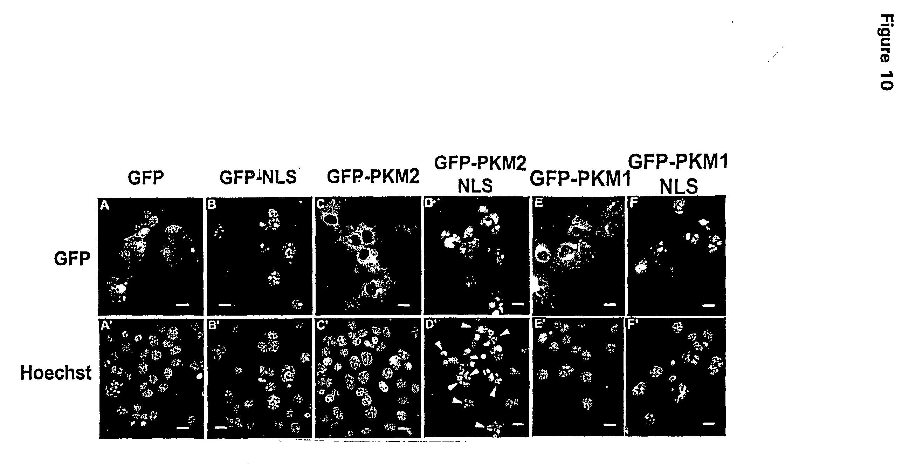

**Fig. 10****.** Effect of nuclear targeted PK-M2 on apoptosis. Cos-7 cells were transfected with-pEGFP (A, A'), pEYFP-NLS (B, B'), pEGFP-PKM2 (C, C') or pEYFP-PKM2-NLS (D, D'), pEGFP-PKM1 (E, E') or pEYFP-PKM1-NLS (F, F') incubated for 48 hours in FCS containing media. Cells were fixed, nuclei were stained with Hoechst and analysed under fluorescent microscope for GFP (A-F) and for nuclear staining (A'-F'). Highly fluorescent, condensed, fragmented nuclei were considered as being apoptotic (arrows).

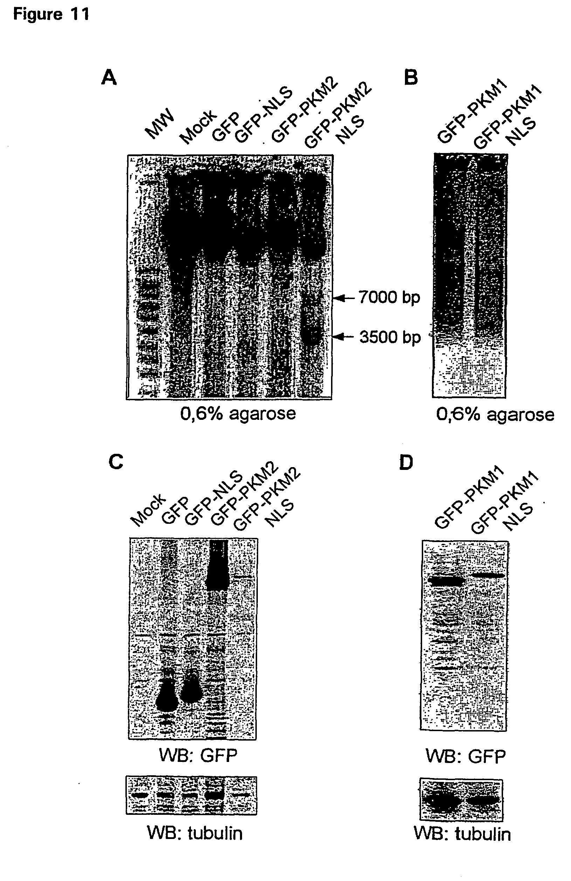

**Fig. 11****.** Detection of DNA degradation. (A and B) Cos-7 cells were transfected with different constructs, incubated for 48 hours in FCS containing media, samples were divided in two parts, and DNA was extracted and analysed on 0,6 % agarose gel. (C and D) second part of the sample was analysed for protein expression. Total cell lysates were loaded on 6-15% gradient gel and analysed in Western-blot with GFP antibody (upper panel). Reprobing the filter with tubulin antibody (lower panel) showed equal loading.

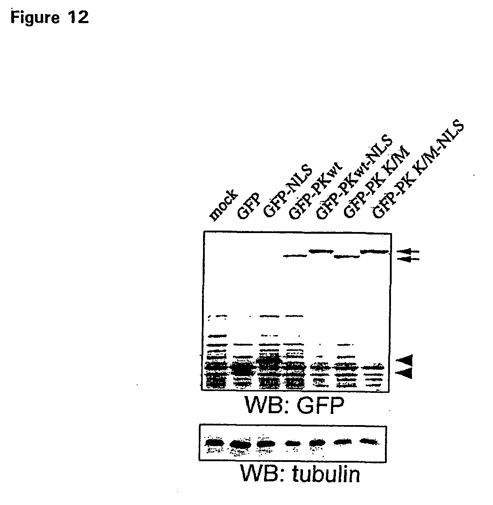

**Fig. 12****.** Expression of kinase deficient pyruvate-kinase in Cos-7 cells. Total cell lysates were loaded on 6-15% gradient gel and analysed in Western-blot with GFP antibody (upper panel, GFP is indicated by arrowheads and GFP-pyruvate kinase M2 fusion proteins by arrows). Reprobing the filter with tubulin antibody (lower panel) showed equal loading.

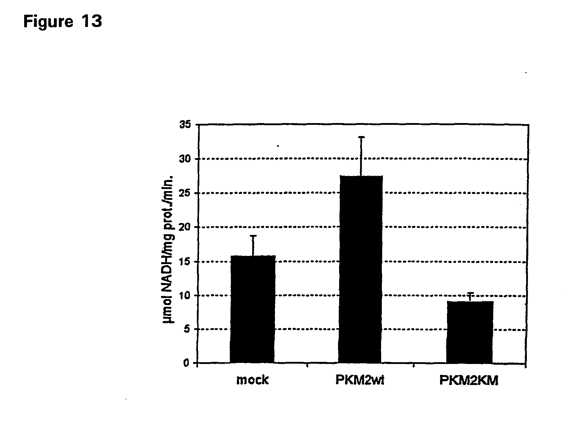

**Fig. 13****.** Pyruvate-kinase activity of Cos-7 cells overexpressing wild type (PKM2wt) or kinase deficient pyruvate-kinase M2 (PKM2KM) Total cell lysates were assayed for pyruvate-kinase activity by LDH coupled assay. (n = 6).

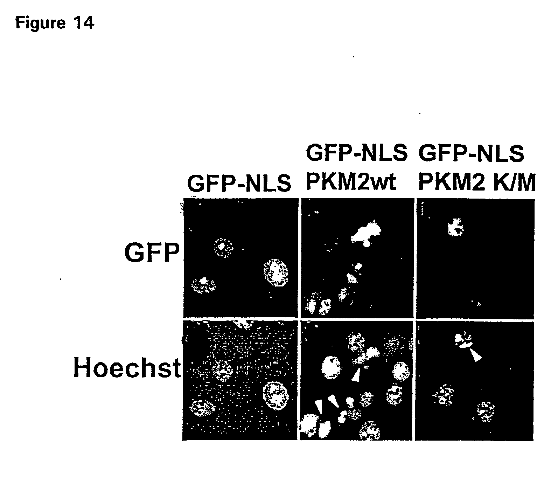

**Fig. 14****.** Effect of nuclear targeted kinase deficient PK-M2 on apoptosis. Cos-7 cells were transfected with pEYFP-NLS (GFP-NLS), pEYFP-PKM2wt-NLS (GFP-NLS PKM2wt) or pEYFP-PKM2 K/M-NLS (GFP-NLS PKM2 K/M) incubated for 48 hours in FCS containing media. Cells were fixed, nuclei were stained with Hoechst and analysed under fluorescent microscope for GFP (upper part) and for nuclear staining (lower part). Highly fluorescent, condensed, fragmented nuclei were considered as being apoptotic (arrows).

### EXAMPLES

The apoptotic effect of a new tumour selective somatostatin analogue was studied; TT-232 and it was found that the compound binds to pyruvate-kinase M2. Pyruvate-kinase M2 isoform was cloned from A431 1 epidermoid carcinoma cell line derived RNA with PCR. Several deletion mutants of the pyruvate-kinase isoform M2 were generated in the eukaryotic expression vector pCDNA3.1 (Invitrogen) as shown in Figure 1.

The effect of the mutants on the induction of apoptosis by TT-232 was investigated in Cos-7 cells transiently overexpressing these mutants. The cells were transfected at 70% confluency with 1µg DNA using the Lipofectamine method. Apoptosis was induced 48 hours after transfection with 25 µM TT-232 and cells were counted 24 hours later. In order to discriminate between living or dying cells methyl-blue exclusion was used (Figure 2).

The overexpression of the N-terminal 100 amino acid of the pyruvate-kinase molecule was sufficient to block the effect of TT-232 somatostatin analogue. This result suggests that pyruvate-kinase, similarly to GAPDH, plays a role in apoptosis. However, since the activity of pyruvate-kinase was not affected by treatment with TT-232 this apoptotic effect is independent of the catalytic activity of the enzyme.

Previously the binding of PK to tubulin was reported (Ovadi et al. 1999) and TT-232 was found to inhibit this interaction. Therefore, the localisation of pyruvate-kinase was studied in detail. NIH3T3 cells were untreated (Figure 3A) or treated with 50 *µ*M TT-232 for 4 hours (Figure 3B), fixed with methanol and subjected to immuno-staining with polyclonal PK-M2 isoform specific and FITC labelled secondary antibody. The nucleus was visualized with propidium-iodide and staining was detected by laser confocal microscopy.

Figure 3 demonstrates that pyruvate-kinase translocates to the nucleus during treatment with the somatostatin analogue TT-232. In order to see whether this effect is a general process during apoptosis, the nuclear translocation was investigated after hydrogen peroxide and UV treatment. Cos-7 cells were transiently transfected with pEGFP-PK-M2 eukaryotic expression plasmid and 48 hours after transfection cells were treated with 1 mM hydrogen peroxide for 4 hours. Cells were treated with 60J/cm² UV radiation and incubated for different times. Cells were fixed with 1 % glutaraldehyde and analysed with fluorescent microscope and InoVision confocal imaging software. Figure 4 shows that the nuclear translocation of the pyruvate-kinase occurs upon treatment with peroxide-peroxide and UV confirming that the translocation of pyruvate-kinase is a general phenomenon.

Finally, we studied the location of the nuclear localisation signal. Therefore several truncated fragments of the pyruvate-kinase were fused to GFP and the localisation of these fragments was studied upon treatment with the TT-232 somatostatin analogue. The transiently transfected Cos-7 cells were untreated or treated with 50 µM TT-232 for 4 hours. Cells were fixed with 1 % glutaraldehyde and the localisation of GFP was detected and analysed with fluorescent microscope and InoVision confocal imaging software. Nuclei were stained with propidium-iodine (Figure 5).

The nuclear translocation was detectable with wild type and ΔC mutant (amino acids 1-420) but not with PK1-334 truncated mutant. Therefore, the pyruvate-kinase protein has a nuclear localisation signal between amino acids 334-420.

We previously isolated pyruvate-kinase M2 as the receptor for the anticancer drug TT-232. To confirm the interaction between pyruvate-kinase M and somatostatin or TT-232 we performed an *in vitro* binding assay with isolated rabbit muscle pyruvate-kinase and ¹²⁵l-somatostatin (SS-14). The binding constant (given as IC₅₀ values) for SS-14 or TT-232 was measured by competition between radio-labelled SS-14 and "cold" peptides (figure 6) and the radioactivity was measured with Phospholmager following gel-electrophoresis of crosslinked proteins.

To investigate the biological role of PK-M2 in the anticancer effect of TT-232 we generated stable 293HEK cells overexpressing HA-tagged wild type PK-M2 and we measured the sensitivity of these cells to TT-232 concentrations. As shown in Figure 7 increasing the amount of pyruvate-kinase protein in cells results increased sensitivity to TT-232, suggesting those pyruvate-kinase plays an important role in the effect of the drug.

In untreated cells, PK-M2 was cytoplasmic. TT-232, hydrogen peroxide and UV induced the nuclear translocation of PK-M2 preceding the appearance of the apoptotic morphology. This result supported our previous data that, like GAPDH, pyruvate-kinase translocates into the nucleus upon apoptotic stimuli. In order investigate further Cos-7 cells were treated, separated into cytosolic and nuclear fractions by NP-40 lysis and analysed with polyclonal PKM2 specific antibody (Weernink et al., 1988) for endogenous PK-M2 by Western-blots. To rule out cross contamination, samples were loaded in triplicate and blotted with PK-M2, Erk-2 or Histone H1 antibodies. As shown in Figure 8, TT-232, hydrogen peroxide and UV treatment induced the translocation of the endogenous PK-M2 into the nucleus with similar kinetics.

However, the enzyme present in the nucleus showed increased electrophoretic mobility, which is probably due to proteolytic cleavage or to dephosphorylation. To test the role of proteases we pre-treated Cos-7 cells with serine-protease (AEBSF, TPCK), caspase (zDEVD) or calpain (ALLN) inhibitor, but were not able to revert this effect. This suggests that none of these proteases are responsible for the apparent processing.

Further, we investigated the role of the M2 isoform in the nucleus. Therefore, PK-M2 and PK-M1 was subcloned into pEYFP-Nuc vector carrying the SV-40 large T antigen nuclear localisation signal fused to GFP and Cos-7 cells were transfected with the different constructs. After 5 hours cells were trypsinized and plated into 96 well and 6 well plates and grown for different times. After 10 hours MTT was added to one of the plates, was measured and this was considered as zero time point. MTT assays demonstrated a significant cell growth inhibition in cells transfected with the nuclear-targeted NLS-PK-M2, while the growth properties of Cos-7 cells carrying GFP, GFP-NLS, PK-M1, NLS-PK-M1 or PK-M2 were unaffected (Fig. 9A and C). To confirm the Expression of the GFP construct, Western-blots with a GFP antibody were carried out from total cell lysates out of the 6 well plates and reprobed with tubulin antibody (Fig. 9B and D).

This result suggested that the nuclear translocation of PK-M2 inhibits cell growth and/or induces apoptosis. We analysed transfected Cos-7 cells stained with Hoechst dye by fluorescent microscopy. Highly fluorescent, condensed or fragmented nuclei that showed patches of compact chromatin were considered as being apoptotic. Figure 10 shows that cells expressing the fusion protein GFP-PKM2, GFP-PKM1 or GFP-NLS-PKM1 showed normal nuclear staining (Fig. 4 C, E, F, C', E' and F') similar to cells carrying GFP or GFP-NLS (Fig. 10A-B'). At the same time, GFP-PK-M2-NLS was found in the nucleus and cells expressing the protein showed apoptotic morphology (Fig. 10D and D').

This result supports that pyruvate-kinase translocation upon apoptotic stimuli is important for the commitment to cell death. This is in conjunction with previous results demonstrating that GAPDH nuclear translocation is involved in apoptosis. In order to test the effect of PK-M2-NLS overexpression on DNA fragmentation, Cos-7 cells were transfected with different constructs, following 48 hours incubation, and DNA was extracted and analysed by agarose gel electrophoresis. Expression of nuclear-targeted PK-M2 induced DNA degradation into distinct larger fragments (3500, 7000 bp) that was different from classical DNA-laddering (Fig. 11).

Since DNA degradation during apoptosis is a multi-step process resulting first large DNA cleavage products than finally oligonucleosomal fragments, we propose that pyruvate-kinase is involved in an early step of DNA degradation, and other factors, like activation of caspases, are necessary for the further processing. Since, overexpressed nuclear targeted PK-M2 was sufficient for cell death independently on the endogenous protein, this effect is likely not due to the inhibition of the glycolysis and the depletion of ATP from cells. This hypothesis is supported by the fact that though GAPDH is related to degenerative conditions in Huntington's disease, the glycolytic activity of the enzyme does not appear to be altered (Kagedal et al., 2001). To test the requirement of the activity of pyruvate-kinase in the nucleus we generated kinase deficient PK-M2 (PKM2K/M) and transfected transiently into Cos-7 cells. First, we tested the expression and processing of the different fusion proteins on Western-blots (Fig. 12).

Next, we measured the pyruvate-kinase catalytic activity from total cell lysates (Fig. 13). Overexpression of the wild type PK-M2 significantly increased the overall PK activity (p> 0,005), while the PK activity in kinase deficient mutant expressing cells was reduced compared to mock transfected cells (p > 0,0001). This suggests that PKM2 K269M mutation, which affects the ATP binding site leads to catalytic inactivation of PKM protein. This is supported by the fact that overexpression of PKM2K/M mutant also interfered with the endogenous PK activity and resulted in decreased overall PK activity.

To investigate the requirement of the activity of pyruvate-kinase in the nucleus we analysed transiently transfected Cos-7 cells for apoptosis by Hoechst staining (Fig. 14).

Taken together pyruvate-kinase was found to be the receptor of a potent antitumour agent with strong apoptotic effect. Further results showed that pyruvate-kinase plays an important role in the apoptotic process, furthermore the enzyme translocates into the nucleus. Nuclear translocation of PK seems to be a general phenomenon, since in oxidative stress (hydrogen-peroxide) or in UV induced apoptosis pyruvate-kinase was found in the nucleus. The nuclear pyruvate-kinase isoform differs from cytosolic pyruvate-kinase in that it exhibits a higher electrophoretic mobility.

It is concluded that like GAPDH, pyruvate-kinase is involved in the apoptotic process and could be a target molecule for the development of drugs against cancer and inflammatory diseases.

### REFERENCES

Tanaka, T., Harano, Y., Sue, F. and Morimura, H. (1967) J. Biochem. (Tokyo) 62, 71-91.
Nowak, T. and Suelter, C. (1981) Mol. Cell. Biochem. 35, 65-75.
Ibsen, K. and Trippet, P. (1974) Arch. Biochem. Biophys. 570-580.
Mallati, A., Yucel, M., Altinors, N. and Gunduz, U. (1992) Cancer Biochem. Biophys. 13, 33-41.
Guminska, M., Stachurska, M.B. and Ignacak, J. (1988) Biochem. Biophys. Acta 966, 207-213.
Saheki, S., Saheki, K. and Tanaka, T. (1982) Biochem. Biophys. Acta 704, 484-493.
Kéri, Gy., et al. (1993a) Biochem. Biophys. Res. Comm. 191, 681-687.
Kéri, Gy., et al. (1993b) Peptide Research 6, 281-288.
Kéri, Gy., et al. (1996), Proc. Natl. Acad. Sci. U.S.A. 93, 12513-12518. Baselga (2000), Semin. Oncol. 27, 27-32.
Monia (2000), Cancer Invest. 18, 635-650.
Vertessy, B.G., Bankfalvi, D., Kovacs, J., Low, P., Lehotzky, A. and Ovadi, J. (1999) Biochem. Biophys. Res. Comm. 254, 430-435.
Weernink, P.A.O., Rijksen, G. and Staal, G.E.J. (1988) FEBS Letters 236, 391-395.
Kagedal, K., Johansson, U. and Ollinger, K. FASEB J. 2001 15, 1592-4.

## Claims

1. A method of identifying novel modulators of apoptosis comprising screening for substances capable of modulating pyruvate kinase M2 activity, wherein the activity comprises a translocation to the nucleus.

2. The method of claim 1 of identifying novel stimulators of apoptosis for use as a pharmaceutical) agent or as a lead structure suitable for the diagnosis, prevention or treatment of a hyperproliferative disease.

3. The method of claim 2 wherein the hyperproliferative disease is selected from tumours, and/or an inflammatory response.

4. Nuclear pyruvate kinase M2 isoform, which exhibits higher electrophoretic mobility compared to the cytosolic pyruvate-kinase isoform.

## Patentansprüche

1. Verfahren zum Erkennen neuer Apoptose-Modulatoren, umfassend die Selektion von Substanzen für die Modulation der M2 Pyruvatkinaseaktivität, wobei die Aktivität eine Translokation zum Kern umfasst.

2. Verfahren nach Anspruch 1 zum Erkennen von neuen Apoptose-Stimulatoren zur Verwendung als pharmazeutisches Mittel oder als Hauptstruktur zur Diagnose, Prävention oder Behanldung einer hyperproliferativen Krankheit.

3. Verfahren nach Anspruch 2, wobei die hyperproliferatve Krankheit ein Tumor und/oder eine entzündliche Reaktion ist.

4. Nukleare M2 Pyruvatkinase-Isoform, welche eine höhere elektrophoretische Mobilität als die cytosolische Pyruvatkinase-Isoform aufweist.

## Revendications

1. Méthode pour identifier de nouveaux modulateurs d'apoptose comprenant le criblage de substances capables de moduler l'activité de la pyruvate kinase M2, dans laquelle l'activité comprend une translocation vers le noyau.

2. Méthode selon la revendication 1, pour identifier de nouveaux stimulateurs de l'apoptose pour l'utilisation comme un agent pharmaceutique ou comme une structure principale adaptée au diagnostic, à la prévention ou au traitement de maladie hypoproliférative.

3. Méthode selon la revendication 2, dans laquelle la maladie hyperproliférative est selectionnée parmi les tumeurs et/ou une réponse inflammatoire.

4. Isoforme de la pyruvate kinase M2 nucléaire, qui montre une mobilité electrophorétique supérieure comparé à l'isoforme de la pyruvate kinase cytosolique.
